# EUROPEAN PATENT SPECIFICATION

(11) **EP 2 341 842 B1**
(45) Date of publication and mention of the grant of the patent: **17.04.2019**
(21) Application number: 09741515.2
(22) Date of filing: 07.10.2009
(51) Int. Cl.: A61B 17/064, A61B 17/122, A61B 17/128, A61B 17/00

(54) **PLICATION DEVICE WITH FORMABLE LINEAR FASTENER FOR USE IN THE DIRECT PLICATION ANNULOPLASTY TREATMENT OF MITRAL VALVE REGURGITATION**
PLIKATIONSVORRICHTUNG MIT FORMBAREM LINEAREM BEFESTIGUNGSELEMENT ZUR VERWENDUNG BEI DER DIREKTEN PLIKATIONS-ANNULOPLASTIEBEHANDLUNG VON MITRALKLAPPENREGURGITATION
DISPOSITIF DE PLICATURE AVEC ÉLÉMENT DE FIXATION LINÉAIRE FORMABLE POUR UNE UTILISATION DANS LE TRAITEMENT D'UNE ANNULOPLASTIE PAR PLICATURE DIRECTE D'UNE RÉGURGITATION DE VALVULE MITRALE

(30) Priority: 06.01.2009 US 349302; 10.10.2008 US 104360 P
(43) Date of publication of application: 13.07.2011
(73) Proprietor: Cardinal Health Switzerland 515 GmbH, 6340 Baar (CH)
(72) Inventor: KREVER, Matthew, Warren NJ 07059 (US); CEDRO, Rudolph, Jr., Clinton NJ 08809 (US); LASH, Timothy, Hillsborough NJ 08844 (US); MAJERCAK, David, Stewartsville NJ 08886 (US); OLSEN, Daniel, H., Califon NJ 07830 (US)
(74) Representative: Prock, Thomas
(86) International application number: PCT/US2009/059770
(87) International publication number: WO 2010/042571

(56) References cited:
- EP-A- 1 607 049
- WO-A1-99/00059
- US-A- 6 139 555
- US-A1- 2007 027 458
- US-B2- 6 716 226

## Description

### FIELD OF THE INVENTION

The present invention relates to a device for treating the vasculature and internal organs of a patient. Particularly, the present invention is directed to a device for treating mitral valve regurgitation in the heart of a patient.

### BACKGROUND OF THE INVENTION

Catheter based devices are used to treat a wide variety of medical problems in a minimally invasive manner. Catheters are used to place and expand angioplasty balloons used to widen veins and arteries narrowed by plaque. Small scaffolds called stents have been introduced into the vasculature using catheter-based systems in order to prevent the restenosis of such vessels. One of the problems that a catheter based device and system could be used to treat in a minimally invasive manner is mitral valve regurgitation, however, no commercially successful device for the treatment of mitral valve regurgitation in such a manner currently exists.

Mitral valve regurgitation is the backflow of blood from the left ventricle into the left atrium due to an improper alignment of the leaflets of the mitral valve thereby causing an imperfect closure of the valve. A gap between the anterior leaflet and posterior leaflet of the mitral valve is created by the improper closure providing a conduit for blood to flow through the mitral valve in a retrograde manner from the left ventricle to the left atrium. This gap may be a congenital defect or may be caused by disease, i.e., ischemic or idiopathic cardiomyopathy and/or intrinsic degenerative disease of components of the mitral valve apparatus. One type of condition, congestive heart failure (CHF), causes the heart to enlarge. In an enlarged heart the walls of the left ventricle are expanded or dilated which causes the papillary muscles to be displaced downward and/or outward resulting in a tethering of the chordae tendineae and subsequent tethering/pulling on the leaflets. Also, with CHF, the mitral annulus is dilated. The combination of the dilated annulus and the tethering on the leaflets prevents the leaflets from closing properly, thereby causing the problematic gap in the mitral valve. The resultant backflow through the mitral valve reduces the efficiency of the heart resulting in a need for the heart to beat faster and/or more forcefully in order to produce the same amount of blood flow. Mitral valve regurgitation may be asymptomatic in some patients but in other patients the reduction in blood flow and the resultant strain on the heart could result in arrhythmias, heart attack and possibly death.

The preferred current treatments for mitral valve regurgitation require open-heart surgery and/or the use of endoscopic techniques that are difficult for the surgeon and potentially dangerous for the patient. In one method of treatment, porcine heart valves or mechanical heart valves are used to replace the damaged or defective mitral valve. Such treatments require the use of open-heart surgery to accomplish the implantation. Such heterologous valves may be used in humans but often wear-out prematurely and additional open-heart surgery is required to replace such valves with additional heterologous or mechanical valves. Mechanical valves have been developed which may also be used as a replacement for a defective mitral valve, however, the implantation of a mechanical valve usually indicates long-term anti-coagulant therapy to prevent clots from developing around the valve that could lead to a dangerous embolism. Long-term anticoagulant treatment causes other problems such as unwanted internal and external bleeding and possibly strokes.

Another open-heart surgical procedure for treating functional mitral valve regurgitation is annuloplasty. In an annuloplasty procedure, a generally "D" shaped annuloplasty ring is implanted on the mitral valve annulus to reduce the size of the stretched mitral valve annulus, most importantly, the septal-lateral dimension and improve closing (or coaptation) of the valve thereby reducing regurgitation. The surgeon surgically attaches, i.e., sews, the annuloplasty ring to the mitral valve on the atrial side of the mitral valve. The annuloplasty ring is sewn to the annulus on a top portion (i.e., the atrial side) of the mitral valve. Once implanted, tissue generally grows over the annuloplasty ring, and a line of contact between the annuloplasty ring and the mitral valve will essentially enable the mitral valve to appear and function as a normal mitral valve by reestablishing coaptation of the mitral valve leaflets but the durability of the effect is variable and may decline within six months after the procedure. Although a patient who receives the annuloplasty ring may be subjected to anti-coagulant therapies, the therapies are not extensive, as a patient is only subjected to the therapies for a matter of weeks, e.g., until tissue grows over the annuloplasty ring.

A second open-heart surgical procedure used in the treatment of degenerative mitral valve regurgitation is the Alfieri stitch procedure which the uses an edge-to-edge suture in the mitral valve. An edge-to-edge stitch is used to stitch together an area at approximately the center of a gap defined between the anterior and posterior leaflets of the mitral valve. Once the stitch is in place, the stitch is pulled in to form a suture that holds the anterior leaflet against the posterior leaflet. By reducing the size of the gap between the anterior leaflet and the posterior leaflet, the amount of leakage through the mitral valve may be substantially reduced. Durability has been a concern for Alfieri procedures done without the addition of an annuloplasty ring. In addition, use of the edge-to-edge procedure is only indicated in certain degenerative pathologies where the primary abnormality or gap between the leaflets is centrally located.

Another method of treating mitral valve regurgitation is the implantation of a ventricular assist device. Such devices are expensive and difficult to implant and require the patient to use anti-coagulant therapy indefinitely. Long-term use of anti-coagulant therapy may result in unnecessary bleeding and strokes. Such ventricular assist devices are, therefore, indicated for use only in patients that would likely not survive without their use and are used to keep patients alive who are candidates for heart transplant surgery. Left ventricular assist devices are a "bridge" therapy rather than a final therapy.

While such invasive surgical procedures have under certain circumstances been shown to be effective in the treatment of mitral valve leakage, invasive surgical procedures often have significant drawbacks. Any time a patient undergoes open-heart surgery, there is a risk of infection. Opening the sternum and using a cardiopulmonary bypass machine has also been shown to result in a significant incidence of both short and long term neurological deficits.

Some minimally invasive procedures have been developed to treat mitral valve regurgitation but to date, none have become commercially successful standard procedures. United States Patent No. 6,619,291 to Hvlaka et al. discloses a minimally invasive method of performing annuloplasty including inserting an implant into a left ventricle and orienting the implant in the left ventricle substantially below the mitral valve. The implant and tissue around the mitral valve are connected and tension is provided to the implant in order to substantially reduce an arc length associated with the mitral valve.

In United States Patent No. 6,718,985 and 7,037,334 to Hvalaka et al. a series of plications near the mitral valve are created by T-bars that are threaded together to reshape the mitral valve. In United States Patent No. 7,166,127 a catheter based system for treatment of mitral valve regurgitation uses a retainers adapted to be secured to the annulus of the mitral valve with flexible tensile members coupled to the retainers. A crimping device deployable through the catheter compresses a crimp onto the flexible tensile members after they are pulled toward one another to reduce the circumferential length of the annulus. In this system the number of permanent implants required in order to achieve an initial effect, and commitment to these implants before success of effect is able to be determined are serious drawbacks.

In United States Patent Application Publication No. 2007/0093857, Rogers et al. describes a device and method for the treatment of mitral valve regurgitation using a minimally invasive procedure in which plications are made proximate the mitral valve of the patient and a retainer is placed to hold the plication.

United States Patent Application No. 2007/0032797 discloses a device for reducing the size of the stomach having a corkscrew-shaped anchor for placement in the gastric wall.

United States Patent Application No. 2007/0025737 to Messerly et al. discloses a surgical retainer having a generally helical shape and a device having jaws for grasping tissue into which the helical retainer may be driven.

United States Patent Application No. 2007/0055335 discloses an electrode probe having a corkscrew-shaped distal tip for use in cardiology applications.

United States Patent Application No. US 2007/0027458 discloses a device for the application of "C" shaped surgical clips.

United States Patent No. US 6,139,555 discloses a surgical clip applier for use in applying ligation as well as for fixing "C" shaped surgical clips or fasteners to a vessel or other tissue at a surgical site.

European Patent Application EP 1 607 049 discloses a surgical clip which has a base portion and two generally parallel, spaced arms extending from the base portion and defining an opening therebetween.

United States Patent Application No. US 2002/0198549 discloses a surgical clip of U-shaped configuration with first and second arms, and a bridge portion therebetween.

The need remains for a device and method for treating mitral valve regurgitation that can be used efficiently and effectively in a minimally invasive procedure and that provides the physician with the ability to know that the procedure has resulted in the desired effect prior to removing the device from the patient thereby reducing the need for and expense of repeat procedures. Such a procedure should provide the physician with the ability to changes the effect on the mitral valve during the procedure before taking an irreversible action.

### SUMMARY OF THE INVENTION

The present invention provides a system which may be used in a method for the treatment of mitral valve regurgitation. The method preferably uses a femoral retrograde approach of crossing the aortic valve. Access to the left ventricle is achieved through the aortic valve using the standard retrograde femoral artery approach utilizing a rounded crossing catheter (CC) preferably with a "J" or pigtail configuration. A deflecting guide catheter is then sent over the crossing catheter into the left ventricle. When the distal end of the deflectable catheter is in the left ventricle the crossing catheter is removed. The deflectable guide is preferably, but need not be, positioned between the papillary muscles with the distal segment lying along the posterior wall of the left ventricle and its tip is pointing towards the underside of the posterior mitral valve annulus. A plication device is then introduced through the deflectable catheter and is advanced out of the distal end of the deflectable catheter and is directed at the underside of the mitral valve, more preferably into the subvalvular groove and positioned so as to be able to grasp and plicate the tissue of the mitral valve at or near the annulus.

A test plication of the mitral valve annulus is created and the appropriateness of the plication is examined using imaging means such as TEE, ICE, TTE or fluoroscopy with or without contrast injection. If the plication is determined to be appropriate then a retainer is applied to the plication to retain the tissue in the plicated state. If the plication is not satisfactory then a retainer is not applied and the jaws of the plication device are released and the plicator is repositioned to plicate a different tissue target at or near the annulus of the mitral valve. Such "test" plications may be repeated a number of times prior to deploying the retainer.

If a single plication and retainer do not sufficiently reshape the mitral valve to correct the regurgitation then the original deflectable guide is repositioned and a second plicator with a retainer is introduced into the delivery guide and positioned and used in the same manner. Alternatively, a multi-retainer plicator can be used to provide the second or third retainers as necessary during the procedure without requiring the removal and reintroduction of the plication device. Once satisfactory changes in the annular geometry of the mitral valve and concomitant reduction in mitral valve regurgitation is achieved then the plication device and the deflectable guide are fully withdrawn and the femoral access site is closed using conventional closing techniques.

Four components comprise the system for percutaneous direct plication annuloplasty. The first is a prolapsable or curved tip crossing catheter preferably having a "J" or pigtail configuration. This may be used with or without a guidewire. In either case the crossing catheter is inserted in a stack or telescoped configuration with the second component, a deflecting guide catheter within which the crossing catheter is initially telescoped or stacked. The deflecting guide catheter is used to provide a means for guiding the plication device into proper position on the underside of the mitral valve preferably at the subvalvular region of the mitral valve at or near the annulus. The third component of the system is a plication device that has an end effector having opposing members at least one of which can be manipulated to open. The plication device is used to grasp tissue and also contains at least one retainer to retain the tissue in the plicated form if desired. In the present invention the retainer is a formable linear retainer. The formable linear design allows for lower profile of the storage of the retainers resulting in the ability to reduce the outer diameter of the device.

The formable linear design enables a more flexible device where the retainers are stored as opposed to the traditional "C" shaped retainers. The formable linear design provides the ability to retain multiple clips in the plication device in addition to providing a retainer that is simple to manufacture.

The present invention is a system for the treatment of mitral valve regurgitation through direct plication annuloplasty of a patient wherein a plication device having a first jaw and second jaw each having a distal end in an opposed arrangement is operable to plicate tissue in the mitral valve of the patient and wherein the plication device comprises at least one formable linear retainer for retaining plications in tissue created by the opposing jaws. The plication device further includes a pusher adapted to engage the proximal end of the formable linear retainer. The pusher is adapted to push the formable linear retainer into the first jaw toward the distal end of the first jaw around the distal end of the first jaw and the distal end of the second jaw and toward the proximal end of the second jaw. The plication device further includes a firing knob connected to a firing control wire adapted to rotate upon rotation of the firing knob in a first direction causing the pusher to move longitudinally and push the formable linear retainer into the plicated tissue. The formable linear retainer further comprises at least one barb disposed on said formable linear retainer for engaging with the plicated tissue.

The formable linear retainer comprises at least one barbed prong angled in a first direction.

The jaws of the plication device may be adapted to engage the proximal end and the distal end of the formable linear retainer and compress said ends together. In a preferred embodiment the plication device contains a guide slot in the proximal end of at least one of the jaws that is adapted to receive the formable linear retainer. The formable linear retainer has a first width at the distal end that is substantially the same width as the guide slot and a second width that at the proximal end that is narrower than the guide slot.

The invention is described in claim 1. Preferred embodiments of the invention are described in the dependent claims.

### BRIEF DESCRIPTION OF THE DRAWINGS

FIGS. 1A and 1B are a flow diagram describing the method of treating mitral valve regurgitation employing the system of the present invention.
FIGS. 2A-H depict the stages of the various steps of the method of treating mitral valve regurgitation employing the system of the present invention.
FIG. 3 depicts the plication regions in the method of treating mitral valve regurgitation employing the system of the present invention.
FIG. 4 is a perspective view of a crossing catheter for use in treating mitral valve regurgitation in accordance with the present disclosure.
FIG. 5 is a cutaway view of a portion of the body of the crossing catheter of FIG. 4.
FIG. 6 is an elevational view of a deflecting guide catheter for use in treating mitral valve regurgitation.
FIG. 7A and 7B are an exploded view and a perspective view respectively of the components of a handle for the deflecting guide catheter of FIG. 6.
FIG. 8 is an elevational view of the body portion of the deflecting guide catheter of FIG. 6.
FIG. 9A and 9B are cross-sectional views of the body portion of the deflecting guide catheter of FIG. 8 taken through lines A and B respectively.
FIGS. 10A-10C are perspective views of the body portion of other embodiments of a deflecting guide catheter for use in treating mitral valve regurgitation
FIG. 11 is an exploded perspective view of another embodiment of the handle and internal components used in a deflecting guide catheter in accordance with the present disclosure.
FIG. 12 is an elevational view of a plication device for use in treating mitral valve regurgitation in accordance with the present invention.
FIG. 13 is an elevational view of the plication device of FIG. 12 with a portion removed to expose the internal components.
FIG. 14A is an elevational view of the plication device of FIGS. 12 and 13 from the shuttle assembly to the distal end.
FIG. 14B is a cross sectional view of the portion of the plication device of FIG. 14A taken through line A-A.
FIG. 14C is an enlarged view of proximal end section D of the cross-sectional view of the portion of the plication device of FIG. 14B.
FIG. 14D is an enlarged view of distal section C of the cross-sectional view of the portion of the plication device of FIG. 14B.
FIG. 14E is an enlarged view of the distal tip section B of the cross-sectional view of the portion of the plication device of FIG. 14B.
FIG. 14F is an enlarged planar view of the distal tip of the plication device of FIG. 14A.
FIG. 14G is a detailed perspective view depicting the coupling of the end-effector control wire to the distal puller wires.
FIG. 14H is a detailed perspective view depicting the coupling of the end-effector control wire to the distal puller wires in an embodiment of the plication device having passive articulation.
FIG. 14I is a perspective view of an embodiment of the jaws in the present invention.
FIG. 14J is a cross-sectional view of the lower jaw of FIG. 14K through line E-E.
FIG. 14K is a side view of the lower jaw of the distal tip of the plication device of FIG. 14I.
FIG. 15A is a cross-sectional view of a formable linear retainer for use in a plication device for use in the treatment of mitral valve regurgitation in accordance with the present invention.
FIG. 15B and FIG.15C are cross-sectional views of plicated tissue retained by a formable linear retainer.
FIG. 15D is a top view of the formable linear retainer of FIG. 15A.
FIGS. 16A-16D are elevational views of the distal end of various embodiments of a plication device in accordance with the present invention.

### DETAILED DESCRIPTION OF THE INVENTION

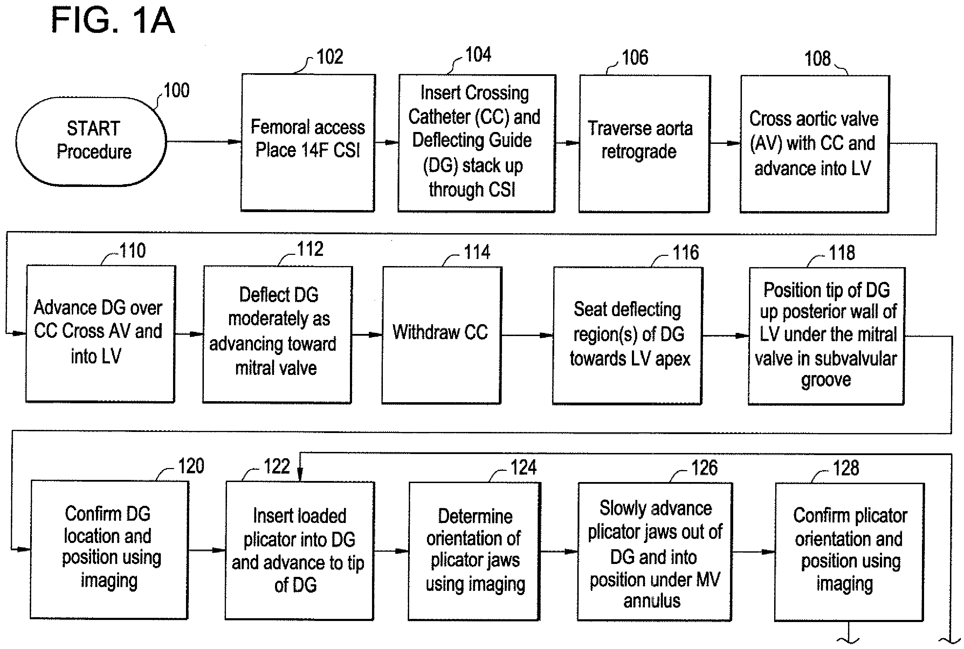

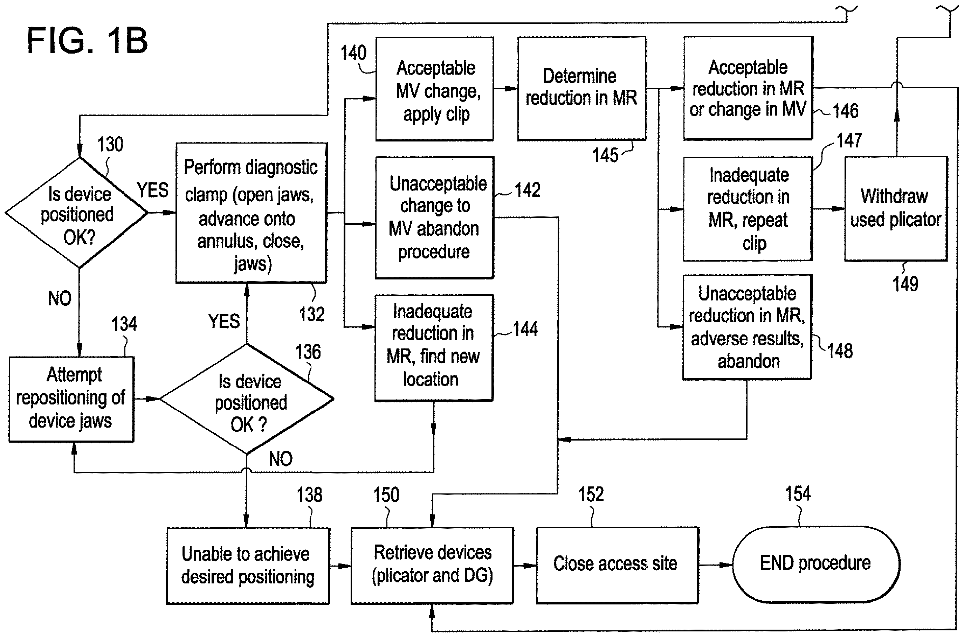

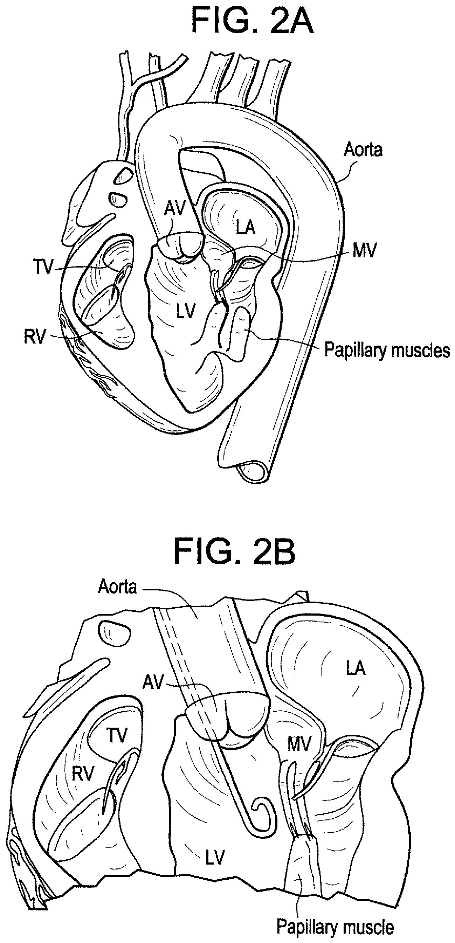

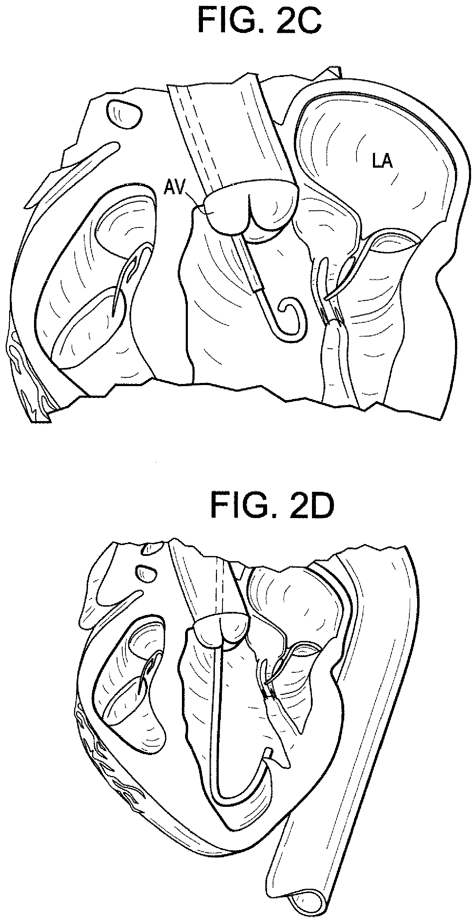

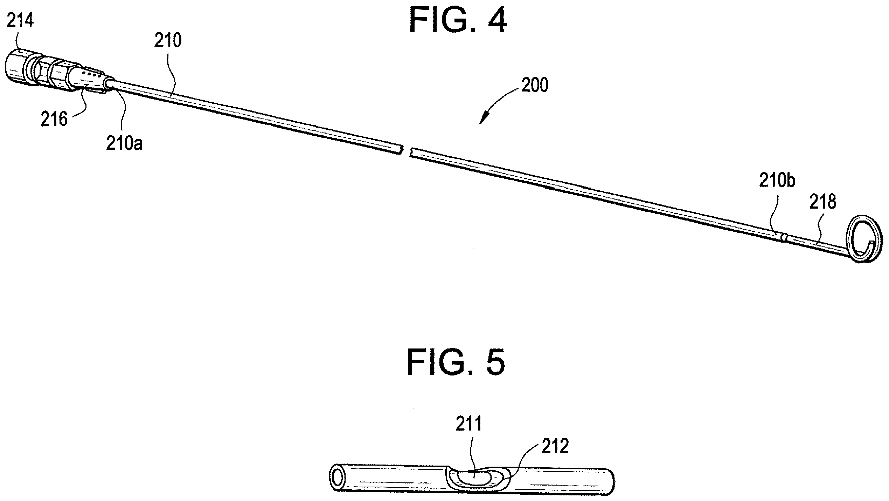

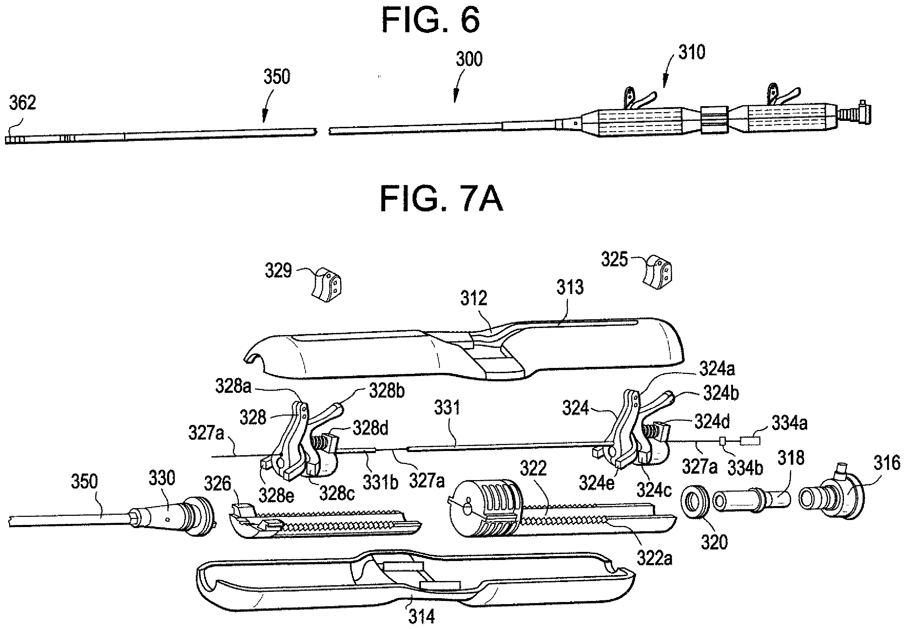

FIG. 1 is a flow diagram depicting a method of providing direct plication annuloplasty to the mitral valve employing the system of the present invention in a heart such as that depicted in FIG. 2A. At step **100** the procedure begins with a puncture for access to the femoral artery using standard techniques. At step **102** the physician or other practitioner places a catheter sheath introducer (CSI) into the femoral access point using standard techniques. Any known CSI may be used in the procedure with the preferable size being approximately 4.667 mm (14 French). At step **104** a crossing catheter, preferably prolapseable or having a curved tip, and a deflecting guide catheter are inserted together in a "stack" formation through the CSI. Alternatively, the deflecting guide catheter is inserted through the CSI without a crossing catheter although the use of a crossing catheter is the preferred method. The crossing catheter is described herein in greater detail with respect to FIGS.4 and 5 below and the deflecting guide catheter is described herein in greater detail with respect to FIGS. 6 to 11. The stacked crossing catheter and deflecting guide catheter are advanced through the arterial system of the patient traversing the aorta of the patient in a retrograde manner at step **106.** At step **108** the aortic valve (AV) is crossed with the crossing catheter and the crossing catheter is advanced into the left ventricle (LV) as depicted in FIG. 2B. At step **110** the deflecting guide catheter is advanced over the crossing catheter through the aortic valve and into the left ventricle as depicted in FIG. 2C. The deflecting guide catheter is deflected in a somewhat retroflexed manner as it is advanced approximately toward the mitral valve at step **112** as depicted in FIG. 2D and the crossing catheter is withdrawn at step **114.**

A guidewire may also be used with the crossing catheter and deflecting guide catheter in a three-element stack inserted in the CSI. If a guidewire is used it is advanced first through the arterial system and over the aortic arch followed by the combined stack of the crossing catheter and the deflecting guide catheter. The guidewire is introduced first through the aortic valve followed by the crossing catheter which is preferably oriented into a position between the papillary muscles although this is not necessary. The procedure then continues as in steps **110** and **112** above with the guidewire removed simultaneously with the crossing catheter at step **114.**

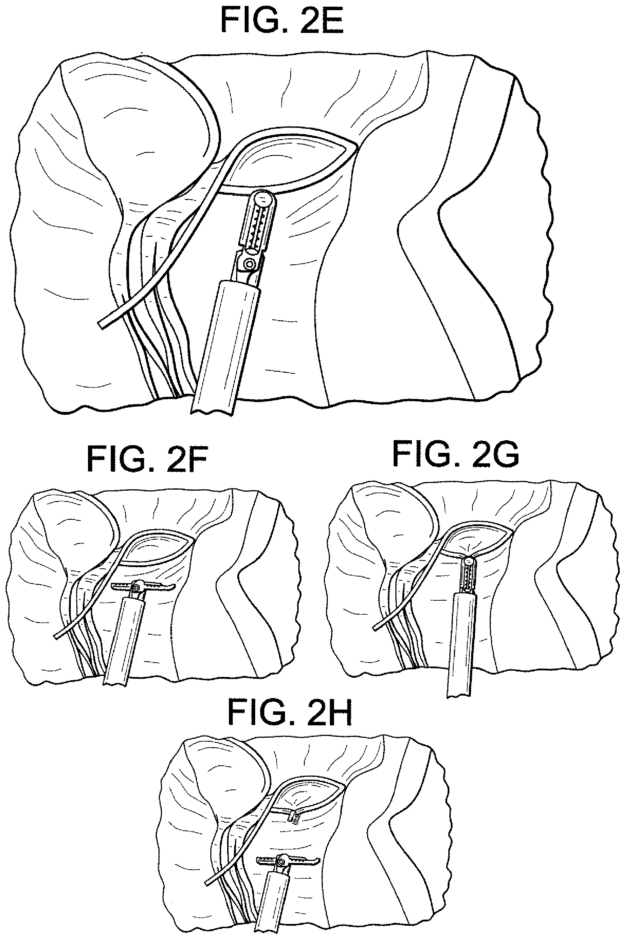

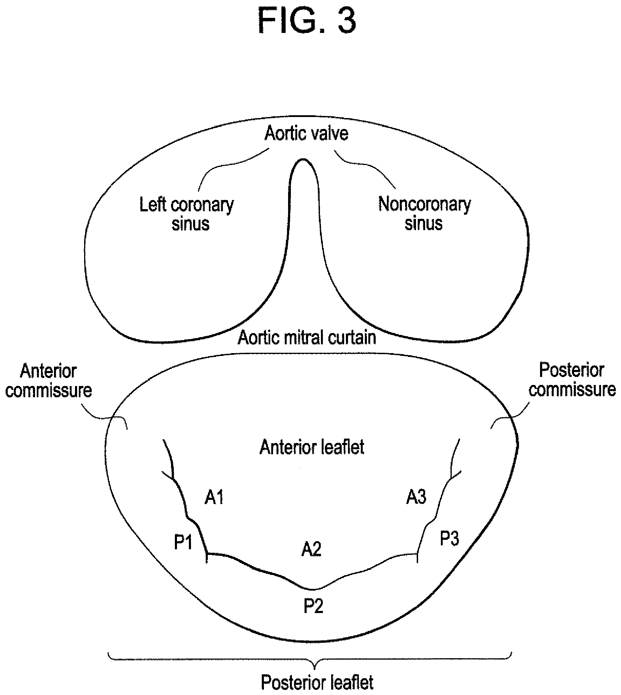

Whether or not a guidewire has been used, the procedure continues with step **116** where a region of the deflecting guide catheter is seated toward the mitral valve in the apex of the left ventricle as in FIG. 2E. At step **118,** the tip of the deflecting guide catheter is advanced up the posterior wall of the left ventricle to a position under the mitral valve, preferably initially placed in the subvalvular groove in the P2 region of the as shown in FIG. 3. The term "annulus" is meant to include regions at or near the annulus. At step **120** the position of the tip of the deflecting guide catheter is confirmed by using an imaging method such as fluoroscopy. If fluoroscopy is used one view maybe sufficient but it is preferable in most cases to use two views to confirm proper placement of the deflecting guide catheter in the P2 region of the mitral valve annulus. P2 is the likely target region for a first retainer although depending on the geometery of the mitral valve the first retainer may be placed in region P1 or region P3. Additional retainers may need to be placed in the same or other regions.

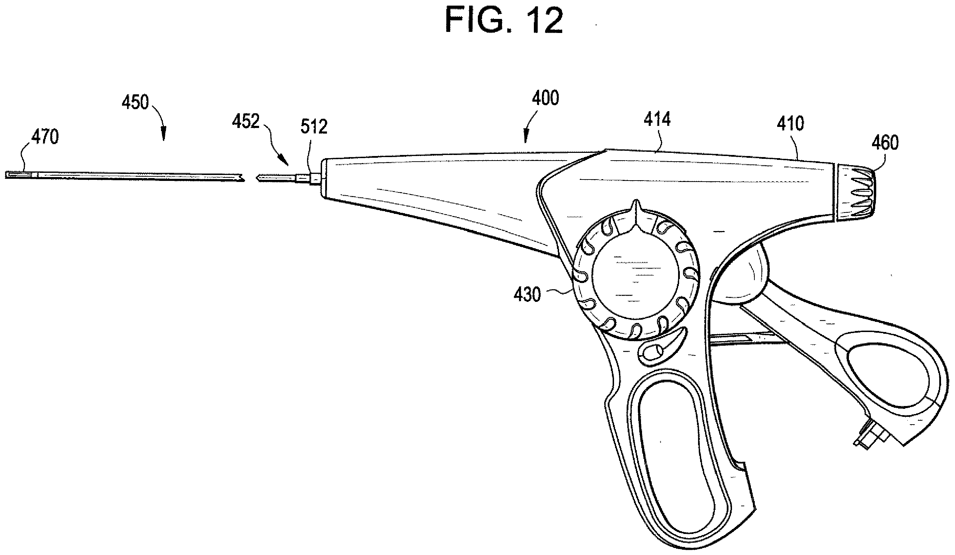

At step **122** a plication device **400** loaded with one or more retainers is inserted into the deflecting guide catheter and advanced to the tip of the deflecting guide catheter. A plication device for use in this method is described in greater detail herein with respect to FIGS. 12 through 14H. At step **124** the rotational orientation of the jaws of the plication device is determined using an imaging method and the jaws are placed in the correct orientation. The preferable rotational orientation for the jaws of the plication device is such that both tips of the jaws once opened would represent a "chord" of the arc defined by the mitral valve annulus when pushed into contact with the annulus. Next, at step **126** the plication device is advanced out of the end of the deflecting guide catheter into position under the annulus of the mitral valve as depicted in FIG. 2E. The orientation and position of the plication device is reconfirmed at step **128** using an imaging method. Again, if fluoroscopy is used as the imaging method, at least one and preferably two views are be used to confirm orientation and placement of the jaws of the plication device. An injection of a known contrast agent either using a separate contrast catheter or through the deflecting guide catheter may be used to help define the line of the annulus as viewed under fluoroscopy. At step **130** a decision is made by the physician whether or not the jaws of the plication device are properly positioned. If the plication device is not correctly positioned then at step **134** an attempt is made to reposition the jaws of the plication device. At step **136** the position of the plication device is evaluated again using an imaging method as described previously and in more detail below. If the plication device is positioned correctly then step **132** and onward are performed as discussed below. If the plication device is not positioned properly after at least one attempt at repositioning at step **134** then step **138** results in a determination that the plication device cannot achieve a desired position and the plication device and deflectable guide catheter are withdrawn from the patient at step **150.**

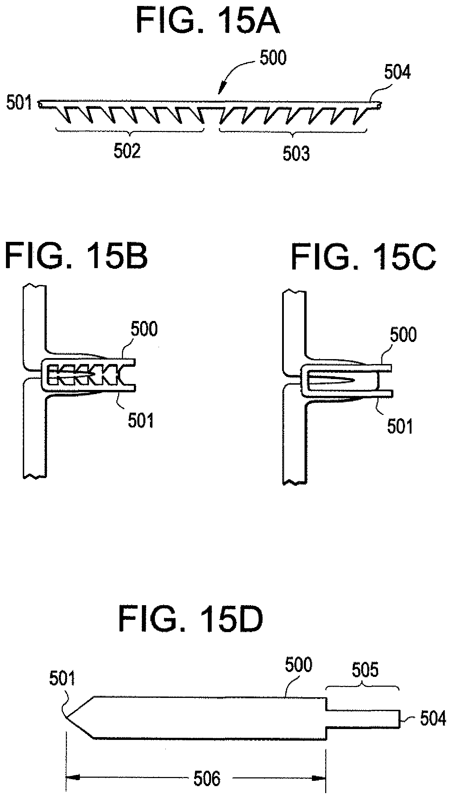

If the jaws are properly positioned, a diagnostic clamp or plication is performed at step **132.** As part of the diagnostic clamping (or plication), the jaws of the plication device are opened as depicted in FIG. 2F, the plication device is advanced onto the tissue of the annulus of the mitral valve and the jaws are closed as depicted in FIG. 2G. The diagnostic plication is evaluated at steps **140, 142** and **144.** If the diagnostic plication results in an acceptable change in the mitral valve annulus and/or an acceptable reduction in mitral valve regurgitation then a retainer is applied using the plication device at step **140** and the plication device is released as depicted in FIG. 2H. Embodiments of a retainer that may be applied to the tissue are described in greater detail herein with respect to FIGS. 15. At step **142,** if the diagnostic plication results in an unacceptable change to the mitral valve then the procedure is abandoned and both the plication device and the deflectable guide catheter are withdrawn from the patient at step **150**. At step **144**, if the diagnostic plication results in an insufficient or inadequate reduction in mitral valve regurgitation (MR) and/or insufficient or inadequate change in the mitral valve then the diagnostic plication is released and an attempt to reposition the jaws of the plication device is performed at step **134**.

If the change to the mitral valve is acceptable and a retainer has been applied, then at step **145** a determination regarding the impact of the plication on the regurgitation of the mitral valve is made using a method of imaging the flow of blood through the valve such as Doppler echocardiograpy. At steps **146, 147** and **148** various decisions are made regarding the procedure and continuation of the procedure. At step **146**, if the determination is made that there has been an acceptable total reduction in mitral valve regurgitation and/or acceptable change in the mitral valve then the procedure branches to step **150** with the retrieval of the plication device and the deflecting guide catheter. If the total change to mitral valve regurgitation is inadequate or insufficient and/or change to the mitral valve is inadequate or insufficient (step **147**) then the plication device currently in use is withdrawn if it is a single retainer device and an additional plication device is inserted and the procedure continues from step **122**. If the plication device is a multi-retainer device then the procedure continues from step **124** without withdrawal of the plication device. If the determination regarding the impact of the plication on mitral valve regurgitation results in a finding of an adverse result at step **148** then the procedure will likely be abandoned and both the plication device and deflecting guide catheter are removed from the patient at step **150**. After removal of the plication device and the deflecting guide catheter, the catheter sheath introducer is removed and the access site is closed at step **152** using known methods.

In the above method various imaging modalities may be used to determine proper placement of the plication device under the mitral valve annulus. Fluoroscopy is one real-time imaging modality that is useful, preferably, where images are taken in at least two planes. Radiopaque markers placed on the distal end of the plication device and/or deflecting guide will aid in determining proper placement. A three-dimensional profile of the plication device can be created using x-ray images acquired in at least two planar projections in real-time. Alternatively, rotational angiographic imaging may be used. Additionally, registering pre-acquired CT or MRI image data with the fluoroscopic image will provide additional anatomic data to the physician to aid proper placement of the plication device and retainer or retainer. Similarly, a three-dimensional real-time ultrasound image acquired in real-time may be registered with the fluoroscopic image.

Another imaging modality useful for this purpose is intracardiac echocardiography (ICE) used to produce an ICE image. The ICE image may be produced by an ICE catheter placed inside one of the chambers of the heart such as the right ventricle, left ventricle, left atrium or the right atrium. Alternatively, the ICE catheter could be placed inside on of the great vessels of the heart of the patient. The ICE catheter may also be placed on the epicardial or pericardial sack surfaces of the heart via a minimally invasive approach such as a sub-xiphoid approach.

No matter the modality used, the images of the mitral valve should be taken synchronized to the cardiac cycle.

Various imaging modalities are also useful in determining whether the plication achieves the desired impact on the function of the mitral valve in real-time or near real-time prior to applying the retainer to the plication. Real-time means that the latency period is acceptable to perform the procedure and is preferably no more than 500 milliseconds. Color Doppler ultrasound imaging may be used for such a purpose with or without an ultrasound contrast agent being administered to the patient. Alternatively, x-ray fluoroscopy could be used in determining the impact of a plication on mitral valve regurgitation by using an x-ray contrast bolus injection into one of the chambers of the heart, preferably the left ventricle. Bi-planar angiographic imaging or intra-chamber optical imaging may also be used. If intra-chamber optical imaging is used it is preferable that the deflecting guide catheter further comprise an optical imaging system particularly one that operates in infrared wavelengths.

Determining a location for the first tissue plication may be based on an optimization plan generated using a three-dimensional functional numerical simulation based on imaging data generated by one or more of the aforementioned imaging method. For example, by analyzing the distribution of annular tissue relative to the location of the primary regurgitant flow through the valve, a primary target for initial plication therapy may be determined. It may be desirable to place the plication at the location of greatest distortion of the annulus due to the pathology of the patient's heart. The generation of the optimization plan may be performed prior to step of inserting the crossing catheter. The generation of the optimization plan may be performed after the step of applying a retainer to the first tissue plication in order to determine the preferred location for subsequent plication or plications.

Alternatively, the plications could be made on the atrial surface if a transseptal approach is used. This can be accomplished by accessing the right atrium using SVC or IVC venous approaches. Then access the left atrium is accomplished using a standard transseptal puncture/access kit such as a Brockenbrough transseptal needle kit. The deflecting guide catheter would then be introduced through the puncture and deflected such that the tip pointed towards the annulus of the mitral valve. The subsequent steps and devices for a plication annuloplasty procedure would then be the substantially the same as set forth above except that the approach is from the atrial side of the mitral valve rather than the underside.

The above method is implemented using a multi-component system comprising a crossing catheter **200,** a deflecting guide catheter **300**, and a plication device **400** containing at least one plication retainer **500**. FIG. 4 is a perspective view of a crossing catheter **200** for use in the procedure described in the present application. Crossing catheter **200** is comprised of a body portion **210** having a proximal end **210a** and a distal end **210b**. Connected to proximal end **210a** are a female luer lock **216** and a Tuohy-Borst hemostasis valve **214**. At the distal end **210b** portion is attached which is preferably a pigtail **218** or has a "J" configuration (not shown). Pigtail **218** is approximately 2.0 centimeters or less in diameter. In FIG. 4 pigtail **218** is attached to body portion **210** at a splice location that is approximately 4 centimeters from the distal end of the device. Pigtail **218** is attached to body portion **210** using heat bonding as the body portion **210** and pigtail **218** re made from the same or similar material. Pigtail **218** is comprised of a polymer, preferably, Pebax ® polyether block amide having a durometer of approximately 55D if comprised of one layer or two layers having durometers of approximately 40D in the outer layer and 55D in the inner layer. Body portion 210 may be comprised of one layer having a durometer between 55D and 72D or may have two layers. If two layers are used the preferred durometers are 70D for the outside and 63D for the inside. The total length of the body portion and pigtail together is approximately 149 centimeters and should extend beyond the deflecting guide catheter when fully inserted into the deflecting guide catheter thus the length of the crossing catheter may vary depending on the length of the deflecting guide catheter used. The location at which the pigtail may be attached to the body portion may also vary from 3 centimeters to approximately 44.5 centimeters from the distal tip of the crossing catheter 200. The crossing catheter may also be comprised of one material from the body portion through the pigtail. In such a case the use of an outer material with a durometer of 55D and an inner material with a durometer of 40D is preferred. A flat wire braid 212 of flat wires of approximately 0.025 mm (.001") by 0.076 mm (.003") may be embedded in the polymer comprising the proximal portion of body portion 210 in order to provide extra stiffness and torqueability. An inner layer 211 of PTFE provide a lubricious inner coating and a separation between the polymer and the inner lumen. The stiffness of the pigtail portion of the crossing catheter is chosen so that a standard guidewire such as the Cordis Emerald® 0.889 mm (0.035") guidewire will open up the pigtail yet will return to the pigtail shape when retracted. Such a guidewire is placed in the guidewire lumen defined by the inner layer 211 of the crossing catheter and should extend through the entire length of the crossing catheter.

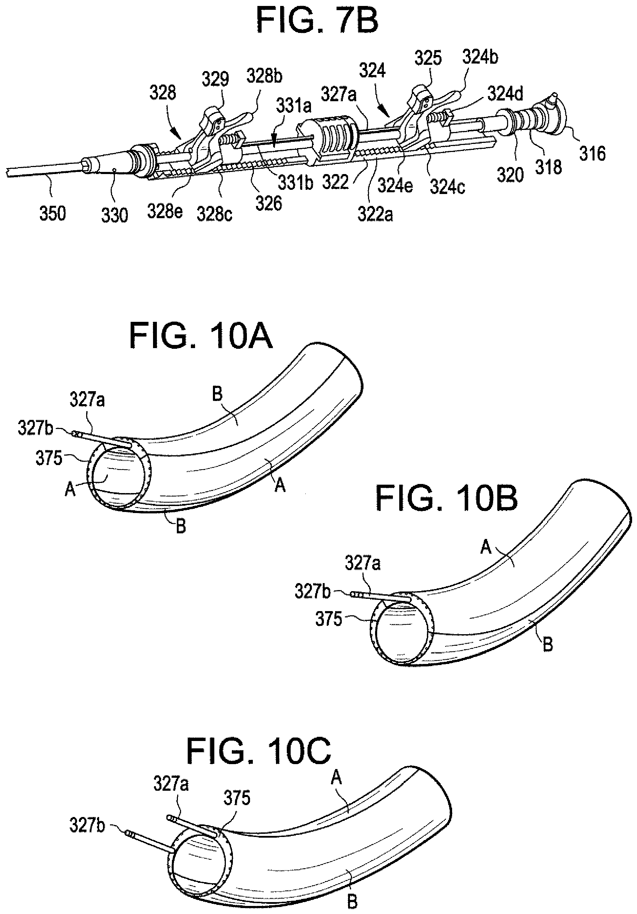

Crossing catheter **200** may be used with or without a guidewire as described above and is preferably used in conjunction with the deflecting guide catheter depicted in FIGS. 6 through 10A-C. Deflecting guide catheter **300** is comprised of a handle **310** and a body portion **350.** FIG. 7A is an exploded view of an embodiment of the handle **310** depicting the internal components of the handle and FIG. 7B is a perspective view of the internal components of handle **310** as assembled. Handle **310** is comprised of upper handle shell **312** and lower handle shell 314 which are made of a durable moldable polymeric material such as polycarbonate or other similar material and are designed to mate with one another in a snap fit arrangement. At the proximal end of handle **310** is a hemostasis valve **316** which is adapted to fit onto the proximal handle tip **318.** Hemostasis valve **316** may be of any known design for such a valve such as a tuohy-borst type valve. Proximal actuator assembly **324** is comprised of a thumb actuator **324a** that is adapted to be inserted through slot **313** in the upper handle shell **312.** Optionally, a two-piece construction with a thumb cap **325** may be used to facilitate assembly if slot **313** is narrow. The thumb actuator **324a** and optional thumb **325** cap are used to cause forward motion in the proximal direction of puller wire **327a.** Such motion is retained as the prong or prongs **324e** biased by spring **324d** around pivot point axel pin **324c** engages the teeth **322a** in proximal rack **322.** Such proximal motion of the proximal actuator assembly **324** and the associated puller wire **327a** causes the deflection of the distal end of the deflecting guide catheter **300.** If the user desires to have distal motion of the proximal actuator assembly **324** then the user pushers release trigger **324b** which counters the bias of spring **324d** thereby releasing prong or prongs **324e** from engagement with the teeth **322a** of the proximal rack **322.** Proximal hypotube **331a** provides a passage way for puller wire **327a** and prevents kinking of the wire. Distal hypotube **331b** is designed to telescope inside hypotube **331a.** At the end of puller wire **327a** are fixedly attached crimp tube **334a** and a floating crimp tube stop **334b** that prevents the crimp tube from being embedded in the proximal end of the actuator assembly. The user may then move the actuator assembly distally thereby changing the deflection of the distal end of the deflecting guide catheter. Movement of the actuator assembly may be made by the physician using something other than his or her thumb and the terms "thumb actuator" and "thumb cap" are not meant to be limiting.

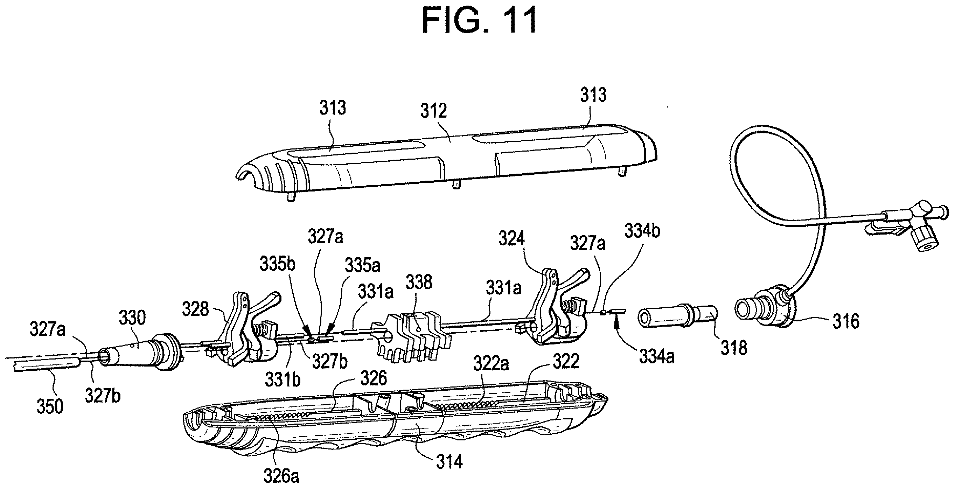

Handle **310** further comprises a distal actuator assembly **328** having a similar thumb actuator **328a,** release trigger **324b,** axel pin **324c,** spring **328d** and prong **328e.** Optional thumb cap **329** is affixed over thumb actuator **328a.** The distal actuator assembly **328** is connected to a second pullerwire **327b** (shown in FIG. 11) that enables the user to cause deflection of the distal end of the deflecting guide catheter. In a preferred embodiment the first and second puller wires are attached (through known methods and means such as welding, brazing or adhesives) to anchor bands **385a** and 385b that are embedded in the distal region 360 of the body portion 350 of the deflecting guide. The puller wires and their respective anchor band connection points may also be arranged so that they are not next to one another (in an axial manner) but so that each provides motion of the distal end in another plane or in the other direction within the same plane. Also, the second puller wire and actuator are not necessary if it is only necessary to provide one type of movement in the deflecting guide catheter. Correspondingly, if greater than two types of deflection are required, additional thumb actuator assemblies coupled to puller wires and anchor bands may be added in a similar manner to the catheter. The second distal actuator assembly has the same components as functions in the same manner as the proximal actuator assembly. The primary difference is that the distal actuator assembly 328 requires a passageway for passage of the first puller wire 327a through the distal assembly which passage is aided by hypotube 331b. The second puller wire 327b ends at the distal end with a similar crimp tube 335a and crimp tube stop 335b. Nose cone 330 provides a transition between the handle shell 312/314 and the proximal region 390 of the body portion 350. Acuator assemblies 324 and 328 and racks 322 and 326 are comprised of a polymeric material such as polycarbonate. Such assemblies could be made of machined or molded metal, such as aluminum, although that would result in a higher cost and weight device. Racks 322 and 326 with teeth 322a and 326a may be separate components or may preferably be molded into the lower handle shell 314 as depicted in the alternative embodiment shown in FIG.11. Handle insert 338 is used as a divider between the two racks 322 and 326 and provides a support for proximal hypotube 331a. Puller wires 327a and 327b are preferably high tensile strength 304 stainless steel (e.g. tensile strength greater than 2068 MPa (300 ksi)) but may also be made of other high strength materials such as MP35N, other stainless steel, or woven fibers such as Kevlar or Vectran.

Puller wires 327a and 327b are preferably a single, solid core high tensile strength 304 stainless steel wire (e.g. tensile strength greater than 2068 MPa (300 ksi)) of approximately 0.203 mm (0.008") in diameter but may also be made of other high strength materials such as MP35N, other stainless steel, or woven fibers such as Kevlar or Vectran. At the distal end of each puller wire is an anchor band 385a or 385b that is embedded in the wall of the catheter body at the point of anchoring. Changing the location of the anchor band along the axial length of the catheter body will change the deflection profile of the deflectable guide catheter.

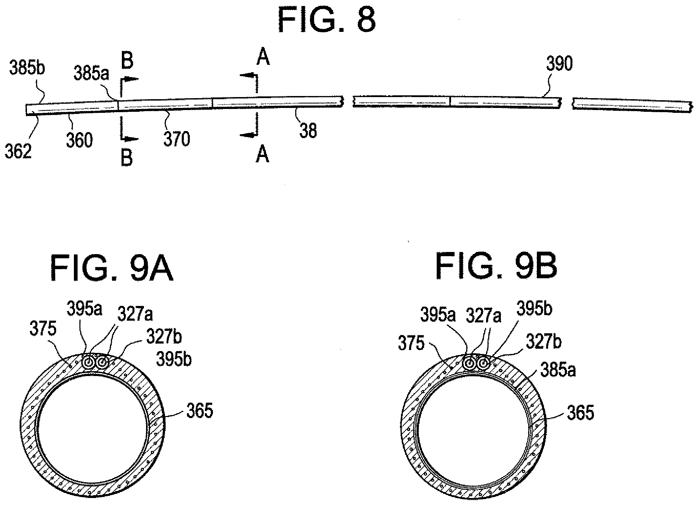

Body portion **350** of deflecting guide catheter **300** is depicted in FIG. 8 and FIGS. 9A and 9B. Body portion is separated into four regions: distal region **360,** intermediate distal region **370,** main intermediate region **380** and proximal region **390.** Distal region **360** at the distal end is approximately 3.5 centimeters in length and is made of a polymeric material such as Pebax® with a durometer of between 25D and 40D and preferably 35D. A radiopaque material such as bismuth subcarbonate is added to the material in distal region **360** to enable the distal region **360** of the deflecting guide catheter **300** appear in fluoroscopy and other imaging procedures. The wall thickness in the distal region **360** is between approximately 0.305 mm (0.012 inches) and 0.356 mm (0.014 inches). The anchor band **385a** for the first puller wire is embedded near the distal end of distal region **360** and the anchor band **385b** for the second puller wire is embedded near the proximal end of distal region **360** or at the distal end of region **370.** The anchor bands are preferable placed between the lubricious liner 365 and the braid 385 although it could be placed above the braid in an alternative embodiment. Each anchor band is made of 304 stainless steel and each puller wire is attached to its respective anchor band using welding or other means for joining metal that is known in the art. The internal diameter of distal region **360** as well as the entire body portion is defined by a lubricious liner **365** preferably PTFE that has an interior diameter of approximately 3.226 mm (0.127 inches) and is approximately 0.051 mm (0.002 inches) thick. The outer diameter of distal region **360** is approximately 4.369 mm (0.172 inches) between the anchor bands and approximately 4.470 mm (0.176 inches) at the location of the distal band. A braid 375 of wires having a diameter between 0.0635 mm (0.0025 inches) and 0.076 mm (0.003 inches) in a 1 over 1 , 1 over 2 under 2 or 2 over 2 pattern is embedded in the polymeric wall of the catheter from the proximal region **390** to the distal region **360.** At the distal end of the distal region **360** of deflecting guide **300** is an extruded atraumatic tip 362 comprised of 33.5% 25D Pebax®, 6.4% 55D Pebax® and 60% bismuth subcarbonate and having a slight taper toward its distal end. The atraumatic tip is optional although preferred in order to avoid tissue damage during insertion in the vessels of the patient.

Intermediate distal region **370** is comprised of the same type of polymeric material but has a higher durometer of between 35D and 55D to provide a stiffer region. Intermediate distal region **370** is between approximately 2.8 and 4.0 centimeters in length and contains the same lubricious liner **365** and wire braid **375** as the distal region. The wall thickness in the intermediate distal region is similarly between 0.305 mm (0.012 inches) and 0.356 mm (0.014 inches) and the outer diameter is approximately 4.369 mm (0.172 inches). Main intermediate region **380** has a slightly smaller outer diameter at 4.216 mm (0.166 inches) but has the same lubricious liner and braid as the other regions. The main difference in this region is the higher durometer of between 55D and 63D for the polymeric material used in order to provide increasing stiffness. The main intermediate region is approximately 20 to 28 centimeters in length, preferably 20 centimeters. Proximal region **390** has a similar composition in that the outer diameter is the same as the immediately prior region. The durometer in this region is increased to approximately 72D providing even greater stiffness and the length of this region is approximately 73 to 88 centimeters, preferably 88 centimeters. The lubricious layer **365** and braid **375** are the same.

From the proximal region **390** through the body portion **350** until the position of first and second anchor bands **385a/385b** run two wire or braid reinforced tubes **395a/395b** of approximately 0.2235 mm (0.0088 inches) in internal diameter which house the first and second puller wires respectively. Various modifications can be made to the deflecting guide catheter if different characteristics are desired. One puller wire, anchor band and reinforced tube could be used instead of two. The braid may be changed to a different size wire and braid type. The polymeric material of the outer body may be varied as depicted in FIGS. 10A-10C. In FIG. 10A materials having two different durometers are used in an alternating fashion. Material A is used in two circumferential portions opposite one another while material **B** is used in two other opposing circumferential portions. The durometer of material A may be greater than the durometer of material **B** or vice versa depending on the deflection characteristics desired. Use of two different durometer materials in such a way provides the benefit of balancing the ability or ease of the catheters to deflect in a particular direction with the requirement for lateral stiffness. In FIG. 10B two circumferential portions of material A and material B are used to provide a certain desired deflection characteristic. In FIG. 10C the use of two different durometer materials is used in conjunction with placement of the puller wires **327a** and **327b** at different places along the circumference of the body portion. In the configuration in FIG. 10C the distal end of the deflecting guide catheter would deflect in two different planes substantially perpendicular to one another. One should note that it is not require to use two different materials or durometer types around the circumference of the outer body in order to get different planes of deflection. The plane of deflection is primarily determined by the relative placement of the puller wire lumens.

The deflecting guide catheter may further comprise a magnetic based location sensor such as those manufactured by Biosense Webster for sensing the location and orientation (six degrees of freedom) of the distal end of the deflecting guide catheter and for providing location information that may be registered with other preaquired or real-time images or otherwise used to depict the location of the distal end of the deflecting guide catheter on a real-time display map of the heart. Systems such as the Carto® system produced by Biosense Webster would be useful for this purpose.

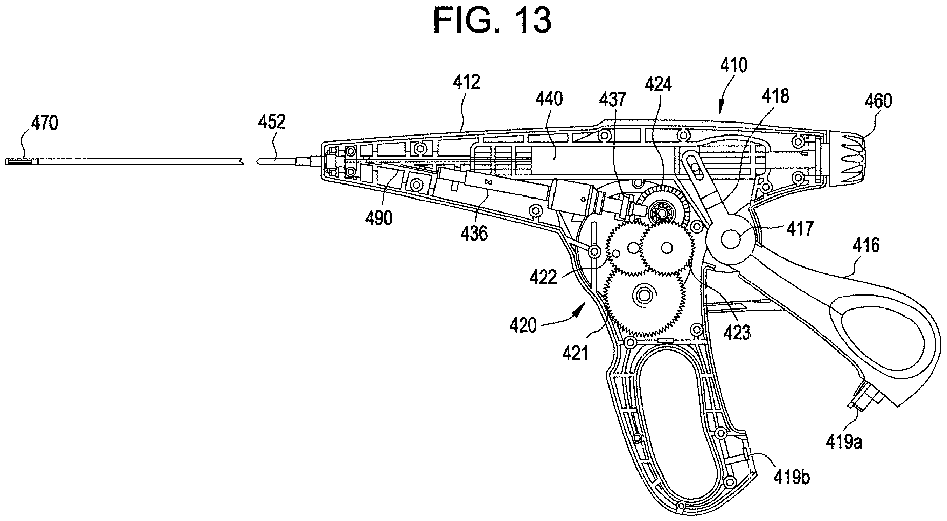

FIG. 12 is an elevational view of a plication device **400** for use in the system for treating mitral valve regurgitation in accordance with the present invention. Plication device **400** is comprised of a handle assembly **410** and a distal assembly **450** having an elongate shaft **452** at the distal end of which are attached a plication assembly with an end effector **520.** FIG. 13 is an elevational view of the internal components of the handle assembly **410.** Handle assembly **410** is comprised of two polycarbonate shell portions - right handle shell **412** and left handle shell **414** that are adapted to house the internal components of the handle assembly. Internal to handle assembly **410** reside crank assembly **420** for advancing a retainer stored in the distal portion of the elongate shaft **452.** The firing assembly **420** is comprised of counter gear **421,** drive gear assembly **422,** idle gear **423,** and crown gear **424.** Firing assembly **420** is coupled to the firing knob **430,** shown in FIG. 12, which is rotatably coupled to left handle shell 414. While not shown, a second firing knob can be disposed on the opposed side of the handle assembly **410** to allow a user to selectively rotate either knob. Either firing knob further comprises a anti-backup leaf spring (not shown) that prevents the knob from turning in the reverse direction and a trigger lockout spring (not shown) that prevents the knob from turning until the trigger is fully closed or engaged. Continuing to refer to FIG. 13, the gears **421, 422, 423** and **424** of firing assembly **420** are configured to rotate in response to rotation of the firing knob **430.** The gears communicate with one another to cause corresponding rotation of pinion assembly **437** and drive shaft **436.** Drive shaft **436** is mated to a proximal end of firing control wire **490.** End cap 460 has a plurality of ridges dispersed around it circumference to aid the grip of the user.

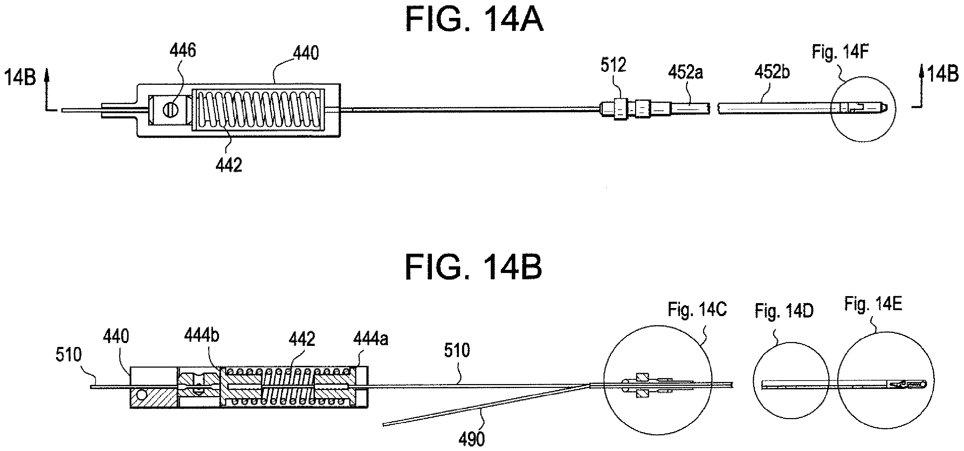

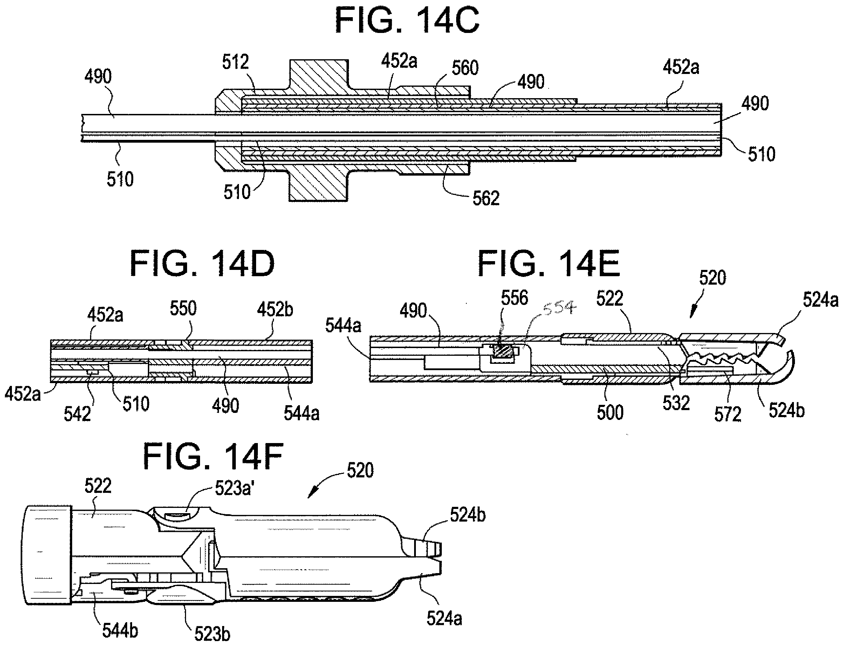

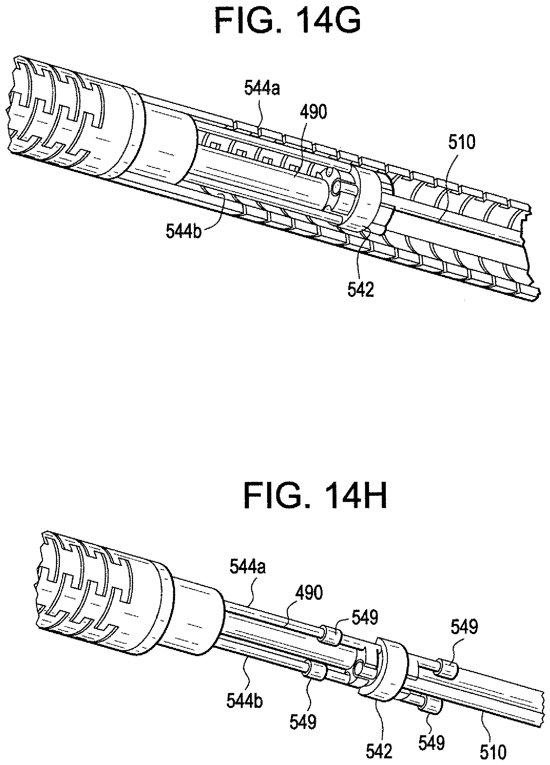

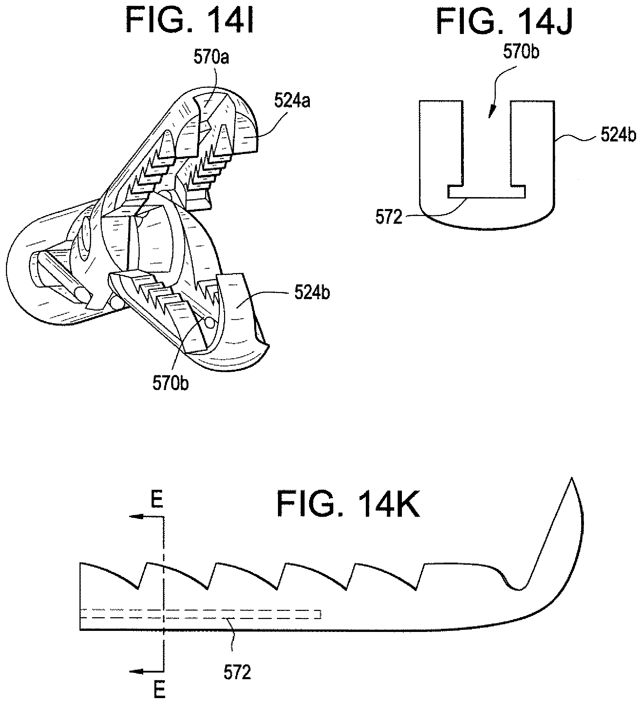

In FIG. 13, the trigger **416** is pivotally mounted within the handle assembly **410** by a pivot pin **417,** and includes a distal portion having a thumb grip formed therein and a proximal extension arm **418.** The trigger **416** also includes a latch **419a** that is adapted to be received in the latch receiver **419b** in the handle assembly to lock the trigger into a closed position. The extension arm **418** is coupled to a shuttle assembly **440** that moves between proximal and distal positions within the housing assembly **410.** The shuttle assembly **440** can have various configurations and it can include various features, such as an overload mechanism. The particular configuration of the shuttle assembly **440** is described in more detail in U.S. Patent Publication No. 2005/0277954. Some of the internal parts of the shuttle assembly 440including spring pin **446,** force limiting spring **442,** spring caps **444a** and **444b** are shown in FIGS. 14A and 14B. As shown in FIG. 13, the shuttle assembly **440** is coupled to a proximal portion of end-effector control wire **510,** which extends through the elongate shaft **452.** The distal end of the end effector control wire **510** mates (preferably by welding) to wire connector **542,** which is shown in FIG. 14D The wire connector **542** is positioned as shown in FIG. 14G proximal to the end effector **520,** i.e., the clevis **522** and jaws **524a** and **524b.** Wire connector **542** is also welded to two parallel pull wires **544a** and **544b** that run from wire connector **542** through nut **550** and terminate in holes at the proximal end of jaws **524a** and **524b** respectively. Thus, wire connector **542** splits the force of end effector control wire **510** into two forces for controlling the opening and closing of the jaws. Other arrangements are possible if, for example, it would be desired to have one fixed jaw and one movable jaw rather than two movable jaws. It is also possible to have some passive articulation of the distal jaws **524a** and **524b** by having the pull wires **544a** and **544b** pass through wire connector **542** as depicted in FIG. 14H and placing a plurality of ferrules **549** in each pull wire **544a** and **544b,** one each proximally and distally of the wire connector **542** proximal end of each wire so that they may translate through the wire connector thereby providing flexibility at the distal tip of the device for improved maneuverability through tortuous anatomical pathways. Distal jaws **524a** and **524b** rotate around pivot point rivots **523a** and **523b** respectively.

The firing control wire **490** extends through the elongate shaft **452** and through a bore formed in the wire connector **542** and is threadably mated to a threaded bore in nut **550.** The distal end of the firing control wire **490** extends into a retainer pusher **554** set in a retainer pusher sleeve **556,** both of which are shown in FIG. 14E. In general, rotation of the firing knob **430** is effective to rotate the firing control wire **490.** Since the firing control wire **490** is threadably mated to the nut **550,** which is fixed between the proximal and distal portions of the elongate shaft **452,** the threaded bore in nut **550** will cause the firing control wire **490** to move distally through the elongate shaft **452,** thereby advancing the retainer pusher **554** in a distal direction. The retainer pusher **554** is positioned proximal a formable linear retainer **500** stored within a garage **532** in the distal portion of the elongate shaft **452,** and thus distal movement of the pusher **554** will advance the retainers **550** through the shaft **452** to position the distal most retainer within the jaws **524a** and **524b** of the end effector **520** as described in greater detail below. A person skilled in the art will appreciate that a variety of other techniques can be used to advance a plurality of retainers through the elongate shaft and to position a retainer within the jaws.

At the proximal end of the elongate shaft **452** is the coil connector **512** which is made of a metal, preferably brass, and is used as a means for connecting the proximal portion **452a** of elongate shaft **452** to the handle assembly. Dual lumen inner sheath **560** has lumens for end-effector control wire **510** and firing control wire **490.** Filler tube connector **562** is used to connect the coil connector **512** to the elongate shaft **452** and is glued to coil connector **512** and elongate shaft **452** using an adhesive glue such as cyanoacrylate. Elongate shaft **452** is broken into proximal shaft section **452a** and distal shaft section **452b.** Proximal shaft section **452a** is preferably nitinol and has a dovetail laser pattern. Distal shaft section **452b** is preferably stainless steel and has a similar dovetail pattern cut through the wall of the shaft. FIGS. 14I-K show a preferred embodiment of the jaws and slots **570a** and **570b** that reside in each jaw **524a** and **524b** respectively and guide the formable linear retainer along its path distally toward the distal end of the plication device and then back proximally through the plicated tissue.

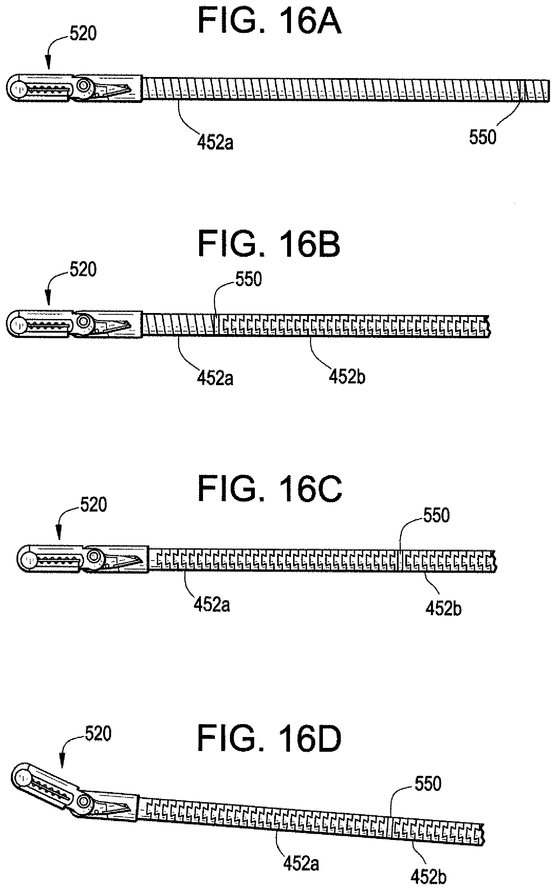

Other patterns could also be used such as a helical cut as shown in FIG. 16A. FIG.16B depicts another variation of the plication device where the proximal shaft section is similar to that above but the nut is placed significantly more distally and the stainless steel distal shaft section with a dovetail pattern is replaced with a helical cut creating a ribbon coil. FIG. 16C depicts the placement of the nut and the dovetail patterns of the proximal and distal shaft portions discussed with respect to FIGS. 14A-F above. FIG. 16D depicts the passively articulating jaws of the alternative embodiment discussed above.

A preferred formable linear retainer **500** for use in the present system and method is shown in FIG. 15. The retainer is comprised of a metal alloy with the preferred embodiment containing at least a trace of an element having an atomic number greater than 53 such as platinum, gold or tantalum to enhance visibility of the retainer under fluoroscopy such as platinum. The retainer may be comprised of a metal or metal alloy such as stainless steel, tantalum, titanium, nitinol, cobalt based alloys and other ductile metals and polymers. The retainer may be coated with a radiopaque layer such as tantalum, gold, platinum or alloys of these materials and tungsten. The formable linear retainer may also be made of a or polymeric material such as one made of poly lactic acid (PLA) and/or poly glycolic acid (PGA).

The retainer may be comprised of a shape memory material such as nitinol and may be manufactured in the formed state so that in the plication device in the linear state it is under compression. The retainer would then take the final state in vivo thereby reducing the need for significant forming of the clip against the jaws. This will reduce the possibility of retainer "spring back" after it is formed by the jaws.

The retainer **500** is substantially linear. The retainer may optionally have a plurality of proximally angled barbs **502** and/or distally angled barbs **503** that prevent the retainer from baking out of the tissue. Such barbs are optional and the proximally angled barbs are preferred if any are used. Preferably the retainer is sharpened at its distal end **501** to increase tissue penetration but remains blunt at proximal end **504** in order to provide a pushing surface for retainer pusher **554.** FIG. 15D depicts the top view of the retainer **500** and shows the wide section **506** that is engages in guide slot **572** and necked down section **505** that has the same width as slot **570b.** As the formable linear retainer is driven forward the wide section is engaged in guide slot **572** which keeps the retainer from being dislodged from the jaw. Once the formable linear retainer has reached its final delivered position the necked down section which has at least the same length as guide slot **572** will fit into slot **570b** thereby permitting release of the formed linear retainer **500.** Prior to applying the retainer to plicated tissue, the retainer is in a linear configuration. Once the decision is made that the plication in the jaws of the plication device is appropriate the distal end of the retainer is pushed into the upper jaw of the plication device and into and/or through the tissue. Continued pushing of the retainer results in the distal end of the retainer being routed around the end portions of the jaws and into the bottom jaw. The end result is the forming of the formable linear retainer into a "reverse" c-shaped retainer wherein the connecting portion of the "C" is embedded within the plicated tissue as shown in FIGS. 15B and FIG. 15C.

If the formable linear retainer has one or more barbs disposed on its surface then it is likely not necessary to perform any final crimping of the exposed ends. If a formable linear retainer without barbs is used then it is may be desired to crimp the exposed ends of the formed retainer. This could be accomplished with the ends of the jaws of the end effector with a slight modification to the jaws to provide slots to engage the tips of the ends **501** and **504** of the retainer

The devices disclosed herein can also be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning and/or replacement, and reassembly. Use of such techniques, and the resulting reconditioned device, are all within the scope of the present disclosure.

The preceding description has been presented with reference to presently preferred embodiments of the invention. Workers skilled in the art and technology to which this invention pertains will appreciate that alterations and changes in the described structure may be practiced without meaningfully departing from the scope of this invention, as defined by the appended claims.

Accordingly, the foregoing description should not be read as pertaining only to the precise structures described and illustrated in the accompanying drawings, but rather should be read consistent with and as support to the following claims which are to have their fullest and fair scope.

## Claims

1. A system for the treatment of mitral valve regurgitation through direct plication annuloplasty of a patient comprising:
a plication device (400) having a first jaw (524a) and second jaw (524b) each having a distal end in an opposed arrangement operable to plicate tissue in the mitral valve of the patient;
wherein the plication device (400) comprises at least one formable linear retainer (500) for retaining plications in tissue created by the opposing jaws (524a and 524b).

2. The system of claim 1 wherein the plication device (400) further includes a pusher (554) adapted to engage the proximal end (504) of the formable linear retainer (500).

3. The system of claim 2 wherein the pusher (554) is adapted to push the formable linear retainer (500) into the first jaw (524a) toward the distal end of the first jaw (524a) around the distal end of the first jaw (524a) and the distal end of the second jaw (524b) and toward the proximal end of the second jaw (524b).

4. The system of claim 2 or 3 wherein the plication device (400) further includes a firing knob (430) connected to a firing control wire (490) adapted to rotate upon rotation of the firing knob (430) in a first direction causing the pusher (554) to move longitudinally and push the formable linear retainer (500) into the plicated tissue.

5. The system of any preceding claim wherein the formable linear retainer (500) further comprises at least one barb (502, 503) disposed on said formable linear retainer (500) for engaging with the plicated tissue.

6. The system of any of claims 1 to 4 wherein the formable linear retainer (500) comprises at least one barbed prong angled in a first direction.

7. The system of claim 5 or 6 wherein the formable linear retainer (500) has at least one radiopaque marker disposed thereon.

8. The system of claim 7 wherein the radiopaque marker disposed on the formable linear retainer (500) is a tantalum microcoil.

9. The system of any of claims 5 to 8 wherein the formable linear retainer (500) is comprised of stainless steel, MP35N, platinum, nitinol, cobalt chromium or alloys thereof.

10. The system of any of claims 5 to 8 wherein the formable linear retainer (500) is comprised of a polymeric material.

11. The system of claim 10 wherein the polymeric material is poly lactic acid (PLA) and/or poly glycolic acid (PGA).

12. The system of any of claims 5 to 11 wherein the formable linear retainer (500) is tapered on the distal end (501) to improve tissue penetration.

13. The system of any of claims 5 to 9 or 12 wherein the formable linear retainer (500) is comprised of a shape memory material manufactured so that it is under compression when implanted into the patient.

14. The system of any preceding claim wherein the jaws (524a, 524b) of the plication device are adapted to engage the proximal end (504) and the distal end (501) of the formable linear retainer (500) and compress said ends (504, 501) together.

15. The system of any preceding claim wherein the plication device (400) contains a guide slot (572) in the proximal end of at least one of the jaws (524a, 524b) that is adapted to receive the formable linear retainer (500).

16. The system of claim 15 wherein the formable linear retainer (500) has a first width at the distal end (501) that is substantially the same width as the guide slot (572) and a second width that at the proximal end (504) that is narrower than the guide slot (572).

## Patentansprüche

1. System zur Behandlung der Mitralklappenregurgitation durch direkte Plikations-Annuloplastie eines Patienten, umfassend:
eine Plikationsvorrichtung (400), welche eine erste Backe (524a) und eine zweite Backe (524b) aufweist, welche jeweils ein distales Ende in einer gegenüberliegenden Anordnung aufweisen, welche betätigbar sind, um Gewebe in der Mitralklappe des Patienten zu falten;
wobei die Plikationsvorrichtung (400) zumindest einen formbaren linearen Halter (500) zum Halten von Plikationen im Gewebe umfasst, welche durch die gegenüberliegenden Backen (524a und 524b) gebildet werden.

2. System nach Anspruch 1, wobei die Plikationsvorrichtung (400) ferner einen Stößel (554) umfasst, welcher ausgebildet ist, um mit dem proximalen Ende (504) des formbaren linearen Halters (500) einzugreifen.

3. System nach Anspruch 2, wobei der Stößel (554) ausgebildet ist, um den formbaren linearen Halter (500) in die erste Backe (524a) zum distalen Ende der ersten Backe (524a) hin, um das distale Ende der ersten Backe (524a) und das distale Ende der zweiten Backe (524b) und zum proximalen Ende der zweiten Backe (524b) hin zu drücken.

4. System nach Anspruch 2 oder 3, wobei die Plikationsvorrichtung (400) ferner einen Auslöseknopf (430) umfasst, welcher mit einem Auslösesteuerdraht (490) verbunden ist, welcher ausgebildet ist, um sich zu drehen, wenn der Auslöseknopf (430) in eine erste Richtung gedreht wird, wodurch der Stößel (554) dazu veranlasst wird, sich in Längsrichtung zu bewegen und den formbaren linearen Halter (500) in das gefaltete Gewebe zu drücken.

5. System nach einem der vorhergehenden Ansprüche, wobei der formbare lineare Halter (500) ferner zumindest einen Widerhaken (502, 503) umfasst, welcher auf dem formbaren linearen Halter (500) zum Eingriff mit dem gefalteten Gewebe angeordnet ist.

6. System nach einem der Ansprüche 1 bis 4, wobei der formbare lineare Halter (500) zumindest eine hakenförmige Zinke umfasst, welche in einer ersten Richtung abgewinkelt ist.

7. System nach Anspruch 5 oder 6, wobei der formbare lineare Halter (500) mindestens einen radioopaken Marker aufweist, welcher darauf angeordnet ist.

8. System nach Anspruch 7, wobei der radioopake Marker, welcher auf dem formbaren linearen Halter (500) angeordnet ist, eine Tantal-Mikrospule ist.

9. System nach einem der Ansprüche 5 bis 8, wobei der formbare lineare Halter (500) aus Edelstahl, MP35N, Platinum, Nitinol, Kobaltchrom oder deren Legierungen besteht.

10. System nach einem der Ansprüche 5 bis 8, wobei der formbare lineare Halter (500) aus Polymermaterial besteht.

11. System nach Anspruch 10, wobei das Polymermaterial eine Polymilchsäure (PLA) und/oder eine Polyglykolsäure (PGA) ist.

12. System nach einem der Ansprüche 5 bis 11, wobei der formbare lineare Halter (500) am distalen Ende (501) verjüngt ist, um die Gewebepenetration zu verbessern.

13. System nach einem der Ansprüche 5 bis 9 oder 12, wobei der formbare lineare Halter (500) aus einem Formgedächtnismaterial besteht, welches so hergestellt ist, dass es komprimiert ist, wenn es in den Patienten implantiert wird.

14. System nach einem der vorhergehenden Ansprüche, wobei die Backen (524a, 524b) der Plikationsvorrichtung zum Eingriff mit dem proximalen Ende (504) und dem distalen Ende (501) des formbaren linearen Halters (500) und zum Zusammendrücken der Enden (504, 501) ausgebildet sind.

15. System nach einem der vorhergehenden Ansprüche, wobei die Plikationsvorrichtung (400) eine Führungsnut (572) im proximalen Ende zumindest einer der Backen (524a, 524b) umfasst, welche ausgebildet ist, um den formbaren linearen Halter (500) aufzunehmen.

16. System nach Anspruch 15, wobei der formbare lineare Halter (500) eine erste Breite am distalen Ende (501), welche im Wesentlichen der Breite der Führungsnut (572) entspricht, und eine zweite Breite am proximalen Ende (504) aufweist, welche schmaler als die der Führungsnut (572) ist.

## Revendications

1. Système pour le traitement de la régurgitation de la valvule mitrale par annuloplastie par plicature directe d'un patient, comprenant :
un dispositif de plicature (400) comportant une première mâchoire (524a) et une deuxième mâchoire (542b), comportant chacune une extrémité distale dans un agencement opposé servant à assurer la plicature des tissus dans la valvule mitrale du patient ;
dans lequel le dispositif de plicature (400) comprend au moins un élément de retenue linéaire formable (500) pour retenir les plicatures dans les tissus formées par les mâchoires opposées (524a et 524b).

2. Système selon la revendication 1, dans lequel le dispositif de plicature (400) inclut en outre un poussoir (554) adapté pour s'engager dans l'extrémité proximale (504) de l'élément de retenue linéaire formable (500).

3. Système selon la revendication 2, dans lequel le poussoir (554) est adapté pour pousser l'élément de retenue linéaire formable (500) dans la première mâchoire (524), vers l'extrémité distale de la première mâchoire (524a), autour de l'extrémité distale de la première mâchoire (524a) et de l'extrémité distale de la deuxième mâchoire (524b), et vers l'extrémité proximale de la deuxième mâchoire (524b).

4. Système selon les revendications 2 ou 3, dans lequel le dispositif de plicature (400) inclut en outre un bouton d'amorçage (430) connecté à un fil de commande de l'amorçage (490), adapté pour tourner lors de la rotation du bouton d"amorçage (430) dans une première direction, entraînant le déplacement du poussoir (554) dans une direction longitude et la poussée de l'élément de retenue linéaire formable (500) dans les tissus plicaturés.

5. Système selon l'une quelconque des revendications précédentes, dans lequel l'élément de retenue linéaire formable (500) comprend en outre au moins un ardillon (502, 503) disposé sur ledit élément de retenue linéaire formable (500) en vue d'un engagement dans les tissus plicaturés.

6. Système selon l'une quelconque des revendications 1 à 4, dans lequel l'élément de retenue linéaire formable (500) comprend au moins un fourchon barbelé incliné dans une première direction.

7. Système selon les revendications 5 ou 6, dans lequel l'élément de retenue linéaire formable (500) comporte au moins un marqueur radio-opaque qui y est disposé.

8. Système selon la revendication 7, dans lequel le marqueur radio-opaque disposé sur l'élément de retenue linéaire formable (500) est une microbobine de tantale.

9. Système selon l'une quelconque des revendications 5 à 8, dans lequel l'élément de retenue linéaire formable (500) est composé d'acier inoxydable, de MP35N, de platine, de nitinol, de chrome-cobalt ou d'alliages de ces substances.

10. Système selon l'une quelconque des revendications 5 à 8, dans lequel l'élément de retenue linéaire formable (500) est composé d'un matériau polymère.

11. Système selon la revendication 10, dans lequel le matériau polymère et un acide polylactique (PLA), et/ou un acide polyglycolique (PGA).

12. Système selon l'une quelconque des revendications 5 à 11, dans lequel l'élément de retenue linéaire formable (500) est effilé sur l'extrémité distale (501) pour améliorer la pénétration dans les tissus.

13. Système selon l'une quelconque des revendications 5 à 9 ou 12, dans lequel l'élément de retenue linéaire formable (500) est composé d'un matériau à mémoire de forme fabriqué de sorte qu'il est sous compression lors de l'implantation dans un patient.

14. Système selon l'une quelconque des revendications précédentes, dans lequel les mâchoires (524a, 524) du dispositif de plicature sont adaptées pour s'engager dans l'extrémité proximale (504) et l'extrémité distale (501) de l'élément de retenue linéaire formable (500) et compriment lesdites extrémités (504, 501).

15. Système selon l'une quelconque des revendications précédentes, dans lequel le dispositif de plicature (400) contient une fente de guidage (572) dans l'extrémité proximale d'au moins une des mâchoires (524a, 524b) adaptée pour recevoir l'élément de retenue linéaire formable (500).

16. Système selon la revendication 15, dans lequel l'élément de retenue linéaire formable (500) a une première largeur au niveau de l'extrémité distale (501) constituant sensiblement la même largeur que celle de la fente de guidage (572), et une deuxième largeur au niveau de l'extrémité proximale, (504) inférieure à celle de la fente de guidage (572).
